Europäisches Patentamt

European Patent Office

Office européen des brevets

(11) Veröffentlichungsnummer: **0 302 439 B1**

(19)

(12) # EUROPÄISCHE PATENTSCHRIFT

(45) Veröffentlichungstag der Patentschrift: **26.01.94**

(21) Anmeldenummer: **88112504.1**

(22) Anmeldetag: **02.08.88**

(51) Int. Cl.⁵: **C07C 253/24**, C07C 255/08

Die Akte enthält technische Angaben, die nach dem Eingang der Anmeldung eingereicht wurden und die nicht in dieser Patentschrift enthalten sind.

(54) **Verfahren zur Herstellung von Acrylnitril.**

(30) Priorität: **07.08.87 DE 3726328**

(43) Veröffentlichungstag der Anmeldung:
**08.02.89 Patentblatt 89/06**

(45) Bekanntmachung des Hinweises auf die Patenterteilung:
**26.01.94 Patentblatt 94/04**

(84) Benannte Vertragsstaaten:
**BE DE ES FR GB IT NL**

(56) Entgegenhaltungen:
**EP-A- 0 057 041**
**DE-A- 3 226 204**
**GB-A- 2 144 049**

(73) Patentinhaber: **BASF plc**
**BASF House**
**151 Wembley Park Drive**
**Wembley Middlesex HA9 8JG(GB)**

(72) Erfinder: **Eichhorn, Hans-Dieter, Dr.**
**47 Davenport Road**
**Cleveland TS15 9TN(GB)**
Erfinder: **Dancey, Martin John**
**68 Roman Road**
**Middlesbrough Cleveland(GB)**
Erfinder: **Herrmann, Guenter, Dr.**
**Haeusserstrasse 51**
**D-6900 Heidelberg(DE)**
Erfinder: **Steen, James Walker**
**41 Cambridge Avenue**
**Middlesbrough Cleveland(GB)**

(74) Vertreter: **Höller, Klaus, Dr. et al**
**BASF Aktiengesellschaft,**
**Patentabteilung ZDX - C 6**
**D-67056 Ludwigshafen (DE)**

**Beschreibung**

Acrylnitril wird in großem technischem Maßstab durch Ammoxidation von Propylen mit Ammoniak und einem molekularen Sauerstoff enthaltenden Gas bei einer Temperatur von 300 bis 600°C in Gegenwart eines Metalloxid-Katalysators der Tellur enthält in der Wirbelschicht hergestellt. Im Verlauf der Reaktion sinkt die Selektivität an Acrylnitril unter vermehrter Bildung von Nebenprodukten durch Verminderung des Tellurgehalts in dem verwendeten Metalloxid-Katalysator. Es hat deshalb nicht an Versuchen gefehlt, den Katalysator zu regenerieren und dessen Aktivität über einen verlängerten Zeitraum aufrecht zu erhalten.

In der DE-PS 2 560 480 wird bereits ein Verfahren beschrieben, bei dem man Metalloxid-Katalysatoren die Tellur enthalten mit einer wäßrigen Lösung oder Suspension von Metalloxid und Tellur imprägniert oder besprüht, anschließend trocknet und dann bei einer Temperatur von 400 bis 850°C calciniert. Mit so behandelten Katalysatoren gelingt es zwar, bei der Ammoxidation von Propylen die Selektivität zu Acrylnitril zu erhöhen, das Verfahren hat jedoch den Nachteil, daß der Katalysator aus der Reaktionszone entfernt werden muß und die Regeneration mehrere Stufen umfaßt. Nach einem anderen, in der Europäischen Patentschrift 57 041 beschriebenen Verfahren, wird die Regeneration von Tellur enthaltenden Metalloxid-Katalysatoren so durchgeführt, daß man Tellurdioxid in Form fester Teilchen dem gewirbelten Katalysator zugibt. Dieses Verfahren hat jedoch den Nachteil, daß das flüchtige Tellurdioxid, ohne sich auf dem Katalysator niederzuschlagen, leicht ausgetragen wird und eine gleichmäßige Beaufschlagung des Katalysators mit Tellurdioxid nicht gewährleistet ist.

GB-A 2,144,049 beschreibt die Regenerierung von Te-haltigen Katalysatoren, die für die Ammoxidation von Propylen geeignet sind, durch Zugabe einer Kombination von Te- und Mo-haltigen calcinierten Verbindungen in fester Form in die Reaktionszone während der Ammoxidation.

Es war deshalb die technische Aufgabe gestellt in einem Verfahren zur Herstellung von Acrylnitril durch Ammoxidation von Propylen den in der Wirbelschicht befindlichen Katalysator ohne Unterbrechung der Reaktion zu regenerieren, wobei die zugeführte Tellurverbindung leicht dosierbar ist und eine gleichmäßige Beaufschlagung des zu regenerierenden Katalysators sichergestellt ist.

Diese Aufgabe wird gelöst in einem Verfahren zur Herstellung von Acrylnitril durch Ammoxidation von Propylen mit Ammoniak und molekularem Sauerstoff oder einem molekularen Sauerstoff enthaltenden Gas bei einer Temperatur von 300 bis 600°C in Gegenwart eines Metalloxid-Katalysators der Tellur sowie mindestens eines der Metalle Eisen, Molybdän oder Antimon enthält, in der Wirbelschicht und Regenerieren des Katalysators, wobei man während der Reaktion dem Katalysator oxidische Tellurverbindungen oder Tellurverbindungen die unter Reaktionsbedingungen oxidische Tellurverbindungen bilden, in gelöster oder suspendierter Form zugibt.

Das neue Verfahren hat den Vorteil, daß es auf einfache Weise gelingt, die Aktivität des Katalysators über längere Zeit aufrechtzuerhalten und die Katalysatoren gleichmäßig mit oxidischen Tellurverbindungen zu beaufschlagen. Weiter hat das neue Verfahren den Vorteil, daß sich das Wirbelverhalten des Katalysatorbetts nicht verändert. Zudem hat das neue Verfahren den Vorteil, daß die Tellurverbindungen leicht und präzise dosierbar sind.

Als Ausgangsstoffe werden Propylen, Ammoniak und molekularer Sauerstoff oder ein molekularen Sauerstoff enthaltendes Gas verwendet. Geeignete molekularen Sauerstoff enthaltende Gase enthalten neben 15 bis 99 Vol%, insbesondere 18 bis 80 Vol.% molekularen Sauerstoff, Inerte, wie Stickstoff, Kohlendioxid sowie Edelgase. Ein geeignetes Gemisch ist beispielsweise Luft. Vorteilhaft hält man ein Molverhältnis von molekularem Sauerstoff : Propylen wie 0,3:1 bis 10:1 und ein Verhältnis von Ammoniak : Propylen von 0,1 bis 5:1 ein.

Die Umsetzung wird bei einer Temperatur von 300 bis 600°C, insbesondere 400 bis 500°C durchgeführt. Zweckmäßig hält man hierbei Drücke von 0,5 bis 4 bar ein.

Die Umsetzung wird in Gegenwart eines Metalloxid-Katalysators, der Tellur sowie mindestens eines der Metalle Eisen, Molybdän oder Antimon enthält, in der Wirbelschicht durchgeführt. Vorzugsweise verwendet man Eisen und Antimon sowie Tellur in oxidischer Form enthaltende Katalysatoren. Vorteilhaft sind die Katalysatoren auf einem Träger, z.B. Siliciumdioxid, Aluminiumoxid, Aluminiumsilikat, Titandioxid oder Gemischen derselben aufgebracht. Besonders bevorzugt sind Katalysatoren der Formel $Fe_{10}Sb_aTe_bMe_cX_dO_e$, wobei Me zumindestens eines der Elemente Vanadium, Molybdän und Wolfram, X zumindestens eines der Elemente Kupfer, Magnesium, Zink, Lanthan, Cer, Aluminium, Chrom, Mangan, Kobalt, Nickel, Wismut, Zinn, Phosphor und Bor, a eine Zahl von 5 bis 60, b eine Zahl von 0,1 bis 10, c eine Zahl von 0,01 bis 10, d eine Zahl von 0 bis 20 bezeichnet und e die Anzahl der Sauerstoffatome darstellt, die den Oxiden entspricht, die durch Vereinigung der beschriebenen Komponenten erhalten werden. Die für die Wirbelschicht geeigneten Tellur enthaltenden Metalloxid-Katalysatoren haben in der Regel eine Partikelgrößenverteilung im Bereich von 5 bis 200 $\mu$m. Die Zugabe der Ausgangsstoffe in die Reaktionszone kann auf unterschiedliche Weise erfolgen. Beispielsweise kann zunächst Luft durch ein Verteilersystem dem Wirbelbettreaktor von unten aufgegeben werden und dadurch die Katalysatorteilchen im fluidisierten Zustand gehalten werden, während Propylen und Ammoniak einzeln oder vermischt durch ein getrenntes Düsensystem in die Reaktionszone eingespeist werden. Um eine optimale Durchmischung zu gewährleisten wird vorteilhaft das Ammoniak-Propylen-Gemisch durch eine Vielzahl von Düsen in das fluidisierte Katalysatorbett zugeführt. Es hat sich dabei als günstig erwiesen, die Zuführung des Ammoniak-Propylen-Gemisches oberhalb der Luftzuführung anzuordnen.

Die Zuführung der Ausgangsstoffe in die Reaktionszone kann jedoch auch auf andere Art und Weise erfolgen. Beispielsweise kann das Ammoniak-Propylen-Gemisch auch mit Luft oder molekularem Sauerstoff angereichert werden oder Luft oder molekularen Sauerstoff enthalten und in verschiedenen radialen Ebenen der Reaktionszone zugeführt werden. Nach einer anderen Arbeitsweise werden Ammoniak, Propylen und Luft bzw. molekularer Sauerstoff vollständig vermischt und der Reaktionszone durch das Verteilersystem vom Boden der Wirbelzone von unten oder auch zusätzlich an verschiedenen radialen Ebenen im Wirbelbett zugeführt werden.

Die Raumgeschwindigkeit definiert als die Masse Katalysator in Gramm pro Volumenmenge an gasförmigen Ausgangsstoffen (Propylen, Ammoniak und Luft oder molekularen Sauerstoff) in $cm^3$ bei Standardtemperaturen und Standarddruck) je Sekunde beträgt in der Regel von 1 bis 15, vorzugsweise von 2 bis 10.

Erfindungsgemäß werden dem Katalysator während der Reaktion oxidische Tellurverbindungen oder Tellurverbindungen, die unter Reaktionsbedingungen oxidische Tellurverbindungen bilden, in gelöster oder suspendierter Form zugegeben, insbesondere in gelöster Form.

Die beim erfindungsgemäßen Verfahren verwendete Lösung enthält zumindestens eine oxidische Tellurverbindung wie ortho-Tellursäure oder Tellursäure. Tellur enthaltende Lösungen können auch durch Auflösen von metallischem Tellur, Tellurmonoxid, Tellurdioxid, telluriger Säure in Salpetersäure hergestellt werden. Eine weitere Möglichkeit ist es z.B. Tellurmetall in Wasserstoffperoxid aufzulösen.

In der Regel enthalten solche Lösungen oxidische Tellurverbindungen in einer Menge von 0,05 bis 250 g/l, insbesondere 0,1 bis 150 g/l, berechnet als Tellur.

Daneben können die tellurhaltigen Lösungen andere Katalysatorwirkstoffe enthalten, die dem Katalysator zugeführt werden sollen. Z.B. kann einem Molybdän enthaltenden Katalysator auf diese Weise eine Molybdänverbindung zugeführt werden. Geeignete Molybdänverbindungen sind beispielsweise Molybdändioxid, Molybdäntrioxid, Ammoniummetamolybdat, Ammoniumparamolybdat, Phosphormolybdänsäure, Bormolybdatsäure oder Molybdatoxalate. Die gelöste Menge richtet sich selbstverständlich nach der Löslichkeit der verwendeten Verbindungen und der Art des Lösungsmittels.

Nach einer anderen erfindungsgemäßen Arbeitsweise wird dem Katalysator während der Reaktion eine Suspension einer oxidischen Tellurverbindung oder mindestens einer Tellurverbindung, die unter Reaktionsbedingungen oxidische Tellurverbindungen bildet, zugeführt. Bevorzugt verwendet man Suspensionen von Tellurdioxid, Telluroxid, Tellurtrioxid oder ortho-Tellursäure, Tellursäure, ferner tellurige Säure. Der Gehalt an Tellurverbindungen beträgt in der Regel 1 bis 200 g/l, insbesondere 2 bis 100 g/l, berechnet als Tellur. Daneben kann die wäßrige Suspension an Tellurverbindungen bei Bedarf auch andere Katalysatorwirkstoffe enthalten, beispielsweise bei einem Molybdän enthaltenden Katalysator Molybdänverbindungen wie Ammoniummetamolybdat, Ammoniumparamolybdat oder Molybdatoxalate; ferner Molybdändioxid oder Molybdäntrioxid.

Es ist auch eine Arbeitsweise möglich bei der die Tellurverbindungen in gelöster Form und die weiteren Zusatzstoffe wie Molybdänverbindungen in suspendierter Form vorliegen oder umgekehrt.

Zur Herstellung einer stabilen Suspension hat es sich als nützlich erwiesen, Suspendierhilfsmittel in wirksamen Mengen zuzusetzen. Geeignete Hilfsmittel sind beispielsweise Tenside wie Alkohole, Polyvinylalkohole, Polyvinylpyrrolidon, Polyethylenglykole.

Die Zuführung der Tellurverbindungen enthaltenden Lösung oder Suspension kann kontinuierlich oder intermittierend erfolgen. In der Regel wählt man die Menge so, daß die Aktivität des Katalysators unter den gewählten Reaktionsbedingungen aufrechterhalten wird. Vorteilhaft hält man 50 bis 200 %, insbesondere 90 bis 150 % der ursprünglichen im frischen Katalysator enthaltenen Tellurmenge aufrecht. Dies läßt sich einfach überprüfen indem man von Zeit zu Zeit dem Wirbelbett Katalysatorproben entnimmt und diese analysiert und die zugeführte Menge an Tellurlösung bzw. Suspension danach richtet.

Als Lösungsmittel oder Suspensionsmittel dient vorzugsweise Wasser oder Salpetersäure. Andererseits ist es jedoch auch möglich, andere geeignete Lösungsmittel wie wäßrige Ammoniaklösung. Acetonitril oder Acrylnitril als Lösungsmittel zu verwenden.

Die Zufuhr der Tellurverbindungen enthaltenden Lösung oder Tellurverbindungen enthaltenden Suspension erfolgt vorteilhaft an mehreren Stellen gleichzeitig, insbesondere dort wo optimale Durchmischungsverhältnisse gegeben sind. Die Lösung bzw. Suspension wird zweckmäßig an den gleichen Stellen zugegeben wie die Ausgangsstoffe. Besonders bewährt hat es sich, wenn man zuzufuhrende Tellurverbindungen enthaltende Lösungen oder Suspensionen direkt dem Wirbelbett zuführt.

Ein erheblicher Vorteil des erfindungsgemäßen Verfahrens besteht darin, daß durch die technisch einfache Art der Beaufschlagung des Wirbelbetts mit Tellurverbindungen enthaltender Lösung bzw. Suspension auch größere unerwartete Tellurverluste ausgeglichen werden können, ohne daß das Wirbelverhalten des Katalysators nachteilig beeinflußt wird oder eine zu hohe Konzentration an Inertstoffen im Reaktor auftritt, wie dies beispielsweise eintritt, wenn Tellur enthaltende Feststoffe gegebenenfalls auf einem inerten Träger dem Katalysatorbett zugeführt werden.

Das nach dem Verfahren der Erfindung erhältliche Acrylnitril wird z.B. zur Herstellung von Polyacrylnitril oder Adipodinitril verwendet.

Das Verfahren nach der Erfindung sei an folgenden Beispielen veranschaulicht. Die in den Beispielen angegebenen Gewichtsteile verhalten sich zu den Volumenteilen wie Kilogramm zu Liter.

Vergleichsbeispiel 1

1000 Gew.-Teile eines Katalysators folgender Zusammensetzung $Fe_{10}Sb_{20}Te_{1,36}Cu_{3,2}Mo_{0,56}W_{0,1}V_{0,1}O_{67,1}$ $(SiO_2)_{55}$, dessen Tellurgehalt auf 88 % des ursprünglichen Wertes abgefallen ist, wird in einem Wirbelbett stündlich mit 635000 Vol.-Teilen (bezogen auf Normbedingungen) einer Gas mischung aus Propylen, Ammoniak und Luft beaufschlagt. Die Gasmischung hat ein molares Verhältnis von Luft : Propylen von 11,0 und ein molares Verhältnis von Ammoniak : Propylen von 1,0. Die Temperatur im Katalysatorwirbelbett beträgt 448°C, der Druck 2,08 bar.

Das aus dem Reaktor austretende Gas wird gaschromatographisch untersucht und die Selektivität der Acrylnitrilbildung und der Propylenumsatzgrad bzw. die Ausbeute an Acrylnitril bestimmt. Die Ergebnisse sind in der nachfolgenden Tabelle aufgeführt.

Vergleichsbeispiel 2

Der Versuch aus Vergleichsbeispiel 1 wird wiederholt mit der Ausnahme, daß ein Katalysator folgender Zusammensetzung $Fe_{10}Sb_{27,5}Te_{1,27}Cu_{3,6}Mo_{0,5}W_{0,2}B_{0,5}P_{0,1}Zn_{0,5}O_{86,9}(SiO_2)_{50}$ eingesetzt wird. Der Katalysator weist noch 94 % seines ursprünglichen Wertes an Tellur auf und zeigt durch längere Einsatzdauer nicht mehr die ursprünglichen Ausbeuten an Wertprodukten.

Die Ergebnisse des Tests sind in der folgenden Tabelle aufgelistet.

Beispiel 1 (erfindungsgemäß)

Der Versuch aus Vergleichsbeispiel 1 wird wiederholt mit der Ausnahme, daß über einen Zeitraum von 14 Stunden pro 1000 Gew.-Teile Katalysator dem Reaktor zusätzlich stündlich 10 Vol.-Teile einer Lösung von ortho-Tellursäure in Wasser zugeführt werden, wobei zur Herstellung der Lösung 12 Gew.-Teile ortho-Tellursäure in 100 Vol.-Teilen Wasser gelöst werden.

Das aus dem Reaktor austretende Gas wird nach einem Zeitraum von 48 Stunden nach Beendigung der Zufuhr der ortho-Tellursäure enthaltenden wäßrigen Losung gaschromatographisch untersucht. Die Ergebnisse sind in der Tabelle enthalten.

Beispiel 2 (erfindungsgemäß)

Der Versuch aus Vergleichsbeispiel 2 wird wiederholt mit der Ausnahme, daß über einen Zeitraum von 13 Stunden pro 1000 Gew.-Teile Katalysator dem Reaktor zusätzlich stündlich 10 Vol.-Teile der Lösung von ortho-Tellursäure in Wasser nach Beispiel 1 zugeführt werden.

Nach einem Zeitraum von 48 Stunden wird das aus dem Reaktor austretende Gas untersucht; die Ergebnisse zeigt die folgende Tabelle.

Beispiel 3 (erfindungsgemäß)

Der Versuch aus Vergleichsbeispiel 2 wird wiederholt mit der Ausnahme, daß über einen Zeitraum von 2 Stunden pro 1000 Gew.-Teile Katalysator der Reaktor mit einer Suspension von 7,5 Gew.-Teilen Tellurdioxid in 20 Vol. Teilen Wasser beaufschlagt wird.

Nach 48 Stunden wird das Reaktoraustrittsgas analysiert.

Vergleichsbeispiel 3

Der Versuch aus Vergleichsbeispiel 1 wird wiederholt mit der Ausnahme, daß der Katalysator wie in Vergleichsbeispiel 2 in frischem ungebrauchten Zustand eingesetzt wird. Die folgende Tabelle zeigt die Ergebnisse des Tests nach 48 Stunden sowie nach 588 Stunden.

Beispiel 4 (erfindungsgemäß)

Der Versuch aus Vergleichsbeispiel 3 wird wiederholt mit der Ausnahme, daß nach etwa 168 Stunden dem Reaktor zusätzlich innerhalb von etwa 3 Stunden eine Tellur-enthaltende Lösung zugeführt wird. Die pro 1000 Gew.-Teilen Katalysator zuzuführende Tellur-enthaltende Lösung wird hergestellt, indem 1,9 Gew.-Teile metallisches Tellurpulver in 10 Vol.-Teilen Wasser suspendiert werden und die auf ca. 95°C erhitzte Suspension langsam mit 6 Vol.-Teilen einer 35 % $H_2O_2$ enthaltenden wäßrigen Lösung versetzt wird. Jeweils nach etwa 168 Stunden seit der letzten Zugabe von Tellurenthaltender Lösung wird die Zugabe wiederholt. Nach einer Gesamtlaufzeit von etwa 690 Stunden wird das Reaktoraustrittsgas analysiert und die Ausbeuten am Wertprodukt bestimmt. Aus dem Reaktor entnommene Katalysatorproben ergeben, daß sich der Tellurgehalt des Katalysators in diesem Zeitraum auf ca. 129 % des ursprünglichen Gehalts erhöht hat.

Beispiel 5 (erfindungsgemäß)

Der Versuch aus Beispiel 4 wird wiederholt mit der Ausnahme, daß die pro 1000 Gew.-Teilen Katalysator zuzuführende Lösung hergestellt wird, indem 1,85 Gew.-Teile metallisches Tellurpulver in einer ausreichenden Menge an 45 % Salpetersäure gelöst werden und die Tellurnitrat enthaltende Lösung mit Wasser versetzt wird, bis sich ein Volumen von 20 Vol.-Teilen ergibt. Nach Beendigung des Versuchs zeigen dem Reaktor entnommene Katalysatorproben, daß sich der Tellurgehalt des Katalysators gegenüber dem ursprünglichen Gehalt um ca. 21 % erhöht hat.

Die Ergebnisse der Vergleichsbeispiele 1 bis 3 sowie der erfindungsgemäßen Beispiele 1 bis 5 zeigen, daß sich ein Verfahren zur Herstellung von Acrylnitril durch Ammoxidation von Propylen unter Verwendung eines Tellur enthaltenden Katalysators mit gleichbleibenden hohen Ausbeuten an Wertprodukten durchführen läßt, wenn der Katalysator im Verlauf der Durchführung der Ammoxidationsreaktion mit einer Tellur enthaltenden Lösung oder Suspension in Kontakt gebracht wird.

Tabelle

Testergebnisse

| | Vgl.-bsp. 1 | Vgl.-bsp. 2 | Bsp. 1 | Bsp. 2 | Bsp. 3 | Vgl.bsp. 3 48 h | Vgl.bsp. 3 nach 588 h | Bsp. 4 | Bsp. 5 |
|---|---|---|---|---|---|---|---|---|---|
| Selektivität für Acrylnitril, % | 76,30 | 76,58 | 77,79 | 78,47 | 78,32 | 78,85 | 76,65 | 78,90 | 78,81 |
| Propylenumsatz % | 94,91 | 95,09 | 95,40 | 95,50 | 95,30 | 95,71 | 95,22 | 95,59 | 95,71 |
| Ausbeute an Acrylnitril, % | 72,42 | 72,82 | 74,21 | 74,94 | 74,64 | 75,47 | 72,99 | 75,42 | 75,43 |

**Patentansprüche**

1. Verfahren zur Herstellung von Acrylnitril durch Ammoxidation von Propylen mit Ammoniak und molekularem Sauerstoff oder einem molekularen Sauerstoff enthaltenden Gas bei einer Temperatur von

6

300 bis 600 °C in Gegenwart eines Metalloxid-Katalysators der Tellur sowie mindestens eines der Metalle Eisen, Molybdän oder Antimon enthält in der Wirbelschicht unter Regenerierung des Katalysators, dadurch gekennzeichnet, daß man während der Reaktion dem Katalysator oxidische Tellurverbindungen oder Tellurverbindungen, die unter Reaktionsbedingungen oxidische Tellurverbindungen bilden, in gelöster oder suspendierter Form zugibt.

2.  Verfahren nach Anspruch 1, dadurch gekennzeichnet, daß man eine Lösung oder Suspension von Tellurverbindungen in Wasser oder Salpetersäure verwendet.

3.  Verfahren nach den Ansprüchen 1 und 2 dadurch gekennzeichnet, daß man 50 bis 200 % des ursprünglichen Tellurgehalts des Katalysators aufrecht erhält.

4.  Verfahren nach den Ansprüchen 1 bis 3 dadurch gekennzeichnet, daß man oxidische Tellurverbindungen oder Tellurverbindungen, die unter Reaktionsbedingungen oxidische Tellurverbindungen bilden, enthaltende Lösung oder Suspension dem Wirbelbett direkt zugibt.

5.  Verfahren nach den Ansprüchen 1 bis 4, dadurch gekennzeichnet, daß die verwendete Lösung oder Suspension von oxidischen Tellurverbindungen oder Tellurverbindungen, die unter Reaktionsbedingungen oxidische Tellurverbindungen bilden, zusätzlich Molybdänverbindungen in gelöster oder suspendierter Form enthält.

6.  Verfahren nach den Ansprüchen 1 bis 5, dadurch gekennzeichnet, daß man einen Eisen und Antimonoxid enthaltenden Katalysator der Telluroxide enthält verwendet.

7.  Verfahren nach den Ansprüchen 1 bis 6, dadurch gekennzeichnet, daß man einen Katalysator verwendet, der der allgemeinen Formel $Fe_{10}Sb_aTe_bMe_cX_dO_e$ entspricht, wobei Me zumindest eines der Elemente Vanadium, Molybdän und Wolfram, X zumindest eines der Elemente Kupfer, Magnesium, Zink, Lanthan, Cer, Aluminium, Chrom, Mangan, Kobalt, Nickel, Wismut, Zinn, Phosphor und Bor bezeichnet, a einer Zahl von 5 bis 60, b einer Zahl von 0,1 bis 10, c einer Zahl von 0,01 bis 10, d einer Zahl von 0 bis 20 und e der Anzahl der Sauerstoffatome der Metalloxide entspricht.

**Claims**

1.  A process for the preparation of acrylonitrile by ammoxidation of propylene with ammonia and molecular oxygen or a gas containing molecular oxygen at from 300 to 600 °C in the presence of a metal oxide catalyst which contains tellurium and one or more of the metals iron, molybdenum and antimony, in a fluidized bed, with regeneration of the catalyst, wherein oxidic tellurium compounds or tellurium compounds which form oxidic tellurium compounds under the reaction conditions, in dissolved or suspended form, are added to the catalyst during the reaction.

2.  A process as claimed in claim 1, wherein a solution or suspension of a tellurium compound in water or nitric acid is used.

3.  A process as claimed in claim 1 or 2, wherein the tellurium content of the catalyst is maintained at from 50 to 200% of the original tellurium content.

4.  A process as claimed in any of claims 1 to 3, wherein the solution or suspension which contains oxidic tellurium compounds or tellurium compounds which form oxidic tellurium compounds under the reaction conditions is added directly to the fluidized bed.

5.  A process as claimed in any of claims 1 to 4, wherein the solution or suspension used of oxidic tellurium compounds or tellurium compounds which form oxidic tellurium compounds under the reaction conditions, additionally contain molybdenum compounds in dissolved or suspended form.

6.  A process as claimed in any of claims 1 to 5, wherein an iron-containing and antimony oxide-containing catalyst which contains tellurium oxides is used.

**7.** A process as claimed in any of claims 1 to 6, wherein the catalyst used is of the formula $Fe_{10}Sb_aTe_bMe_cX_dO_e$, where Me is one or more of the elements vanadium, molybdenum and tungsten, X is one or more of the elements copper, magnesium, zinc, lanthanum, cerium, aluminum, chromium, manganese, cobalt, nickel, bismuth, tin, phosphorus and boron, a is from 5 to 60, b is from 0.1 to 10, c is from 0.01 to 10, d is from 0 to 20 and e is the number of oxygen atoms of the metal oxides.

**Revendications**

**1.** Procédé de préparation d'acrylonitrile par ammoxydation de propylène avec de l'ammoniac et de l'oxygène moléculaire au un gaz contenant de l'oxygène moléculaire, à une température de 300 à 600°C, en présence d'un catalyseur à base d'oxydes métalliques qui contient du tellure ainsi qu'au moins l'un des métaux fer, molybdène ou antimoine, en lit fluidisé avec régénération de catalyseur, caractérisé en ce que, pendant la réaction, on ajoute au catalyseur des composés oxydés du tellure ou des composés du tellure qui forment dans les conditions de la réaction des composés oxydés du tellure, sous forme dissoute ou en suspension.

**2.** Procédé selon la revendication 1, caractérisé en ce qu'on utilise une solution ou une suspension de composés du tellure dans de l'eau ou de l'acide nitrique.

**3.** Procédé selon l'une ou l'autre des revendications 1 et 2, caractérisé en ce que l'on maintient 50 à 200% de la teneur en tellure initiale du catalyseur.

**4.** Procédé selon l'une quelconque des revendications 1 a 3, caractérisé en ce qu'on ajoute directement au lit fluidisé la solution ou la suspension contenant des composés oxydés du tellure ou des composés du tellure qui forment dans les conditions de la réaction des composés oxydés du tellure.

**5.** Procédé selon l'une quelconque des revendications 1 à 4, caractérisé en ce que la solution ou suspension utilisée de composés oxydés du tellure ou de composés du tellure qui forment dans les conditions de la réaction des composés oxydés du tellure, contient en plus des composés du molybdène sous forme dissoute ou en suspension.

**6.** Procédé selon l'une quelconque des revendications 1 à 5, caractérisé en ce qu'on utilise un catalyseur contenant du fer et de l'oxyde d'antimoine qui contient de l'oxyde de tellure.

**7.** Procédé selon l'une quelconque des revendications 1 à 6, caractérisé en ce qu'on utilise un catalyseur qui correspond à la formule générale $Fe_{10}Sb_aTe_bMe_cX_dO_e$ dans laquelle Me représente au moins l'un des éléments vanadium, molybdène et tungstène, X représente au moins l'un des éléments cuivre, magnésium, zinc, lanthane, cérium, aluminium, chrome, manganèse, cobalt, nickel, bismuth, étain, phosphore et bore, a représente un nombre de 5 a 60, b représente un nombre de 0,1 à 10, c représente un nombre de 0,01 à 10, d représente un nombre de 0 à 20 et e représente le nombre d'atomes d'oxygène de l'oxyde métallique.